# EUROPEAN PATENT APPLICATION

(11) **EP 0 798 562 A2**
(43) Date of publication of application: **01.10.1997**
(21) Application number: 97302099.3
(22) Date of filing: 26.03.1997
(51) Int. Cl.: G01N 33/58, C12N 9/02

(54) **Multiple immunoassays**

(30) Priority: 26.03.1996 JP 94702/96
(71) Applicant: KIKKOMAN CORPORATION, Noda-shi, Chiba-ken 278 (JP)
(72) Inventor: Tatsumi, Hiroki, Noda-shi, Chiba-ken 278 (JP); Fukuda, Satoshi, Noda-shi, Chiba-ken 278 (JP); Kikuchi, Mamoru, Noda-shi, Chiba-ken 278 (JP); Koyama, Yasuji, Noda-shi, Chiba-ken 278 (JP)
(74) Representative: Woods, Geoffrey Corlett

(57) **Abstract**

An immunoassay using an enzyme as a label, wherein two or more kinds of luciferase are used as the label to determine two or more substances. Each luciferase emits light of a different respective wavelength when acting upon a luciferin substrate.

## Description

The present invention relates to a multiple immunoassay for simultaneously determining two or more substances to be determined.

An immunoassay is a method for determining a trace substance by binding a specific antibody or antigen via antigen-antibody reaction to the corresponding antigen or antibody as the subject of determination.

A labeling method in such immunoassays is a method using a label to determine a substance in a sample.

In conventional immunoassays, there is no established method to detect two or more substances simultaneously by a luminescent method which is superior in sensitivity to a coloration method, fluorescent method etc.

The object of the present invention is to provide a multiple immunoassay using an enzyme as a label wherein two or more substances are determined simultaneously by a luminescent method.

As a result of their eager research, the present inventors found that two or more substances can be detected simultaneously in an immunoassay by use of two or more kinds of luciferase capable of emitting different colors of light respectively by catalyzing luciferin as labels, to complete the present invention.

That is, the present invention is a multiple immunoassay using an enzyme as a label wherein two or more kinds of luciferase are used as the label to determine two or more substances.

According to the immunoassays of the present invention, two or more substances can be determined so the present invention is extremely useful.

In the accompanying drawings:

FIG. 1 shows a scheme for constructing recombinant plasmid pHLf203 DNA.

FIG. 2 shows a scheme for constructing recombinant plasmid pHLf213 DNA.

FIG. 3 shows a photograph made monochrome by a computer from a colour slide of dot plots using complexes of two kinds of biotinated luciferase/streptavidin/biotinated antibodies.

Hereinafter, the present invention is described in detail.

The luciferase may be any wild-type Coleoptera luciferases isolated from Luciola cruciata, Luciola lateralis, Photinus pyralis etc. Some of these wild-type Coleoptera luciferases can emit light of wavelengths around 560 nm (olive green color) by catalyzing luciferin.

The luciferase may also be any mutant Coleoptera luciferases. The mutant luciferase can be obtained by subjecting to random mutation a wild-type Coleoptera luciferase gene derived from e.g. Luciola cruciata, Luciola lateralis or Photinus pyralis to prepare a mutant Coleoptera luciferase gene, then culturing a microorganism rendered capable of producing the mutant luciferase by transformation or transduction with a recombinant vector carrying said mutant Coleoptera luciferase gene, and recovering it from the culture (see Japanese Patent Application LOP Publication No. 285,683/1991 or US Patent No. 5,219,737). An example is a mutant luciferase having the amino acid sequence of the luciferase of Luciola cruciata in which one of the following changes appears: serine is replaced by asparagine at amino acid 286, glycine is replaced by serine at amino acid 326, histidine is replaced by tyrosine at amino acid 433 or proline is replaced by serine at amino acid 452, and such mutant luciferases can emit light of wavelengths of 607 nm (orange), 609 nm (red), 612 (red), and 595 nm (orange) respectively by catalyzing luciferin. Furthermore, the mutant luciferase may be a mutant Coleoptera luciferase obtained by mutating a wild-type Coleoptera luciferase by site-specific mutation (Kunkel, T. A. et al., Methods in Enzymology, 154, 367-382 (1987)).

The present invention comprises use of two or more kinds of luciferase described above, preferably two or more kinds of luciferase capable of emitting different colors of light respectively by catalyzing luciferin.

The immunoassay of the present invention comprises use of antigen or antibody labeled with such luciferases, that is, luciferase/antigen or luciferase/antibody complexes. The luciferase/antigen (or antibody) complexes can be produced as a fusion protein by a microorganism transformed or transduced with a recombinant vector containing a luciferase gene bound to an antigen (or antibody) gene. The luciferase/antigen (or antibody) complexes can also be produced by biotinating both luciferase and antigen (or antibody) and then linking them via avidin or streptavidin. Alternatively, the luciferase/antigen (or antibody) complexes can be produced by biotinating either luciferase or antigen (or antibody), while binding another to avidin or streptavidin, and linking them.

To biotinate luciferase, antigen or antibody, it is possible to employ e.g. chemical modification (see Methods in Enzymology, 184, 123-173 (1990)), genetic manipulation means (see Weiss, E. et al., Protein Expression and Purification, 5, 509-517 (1994), Japanese Patent Application No. 98857/1995, and US Patent Application No. 08/460,934) etc. To bind avidin or streptavidin directly to a protein, it is possible to employ e.g. chemical modification (see Methods in Enzymology, 184, 174-207 (1990)), genetic manipulation means (see Japanese Patent Appln. LOP Publication No. 289,264/1995, and Airenne, K. J. and Kulomaa, M. S., Gene, 167, 63-68 (1995)) etc.

In this manner, two or more kinds of luciferase emitting different colors of light can be bound respectively to different antigen or antibody to give two or more kinds of complex.

The antigen is not limited and includes e.g. enzymes, hormones, cytokines, biologically active substances, agrochemicals, toxins, microorganisms etc. The antibody is not limited and includes e.g. mammalian IgG, mammalian IgM, avian IgY etc.

Antibody-binding proteins such as protein A etc. or biotin-binding proteins such as streptavidin etc. can be labeled for use as a secondary label.

The immunoassay of the present invention is carried as follows. First of all, two or more kinds of substances to be determined, that is, antigens or antibodies, are immobilized onto a carrier. For example, these substances can be immobilized on a nitrocellulose membrane or PVDF membrane directly or by Western blot technique (Sambrook J. et al., Molecular Cloning, second edition, 18.60-18.75 (1989)), or on wells in a microtiter plate. Then, the membrane or wells are washed with cleaning solution, and two or more kinds of luciferase/antigen or luciferase/antibody complexes are added to effect specific binding between the antigens and luciferase/antibody complexes or the antibodies and luciferase/antigen complexes. After washing, a substrate solution containing e.g. luciferin and ATP is added. Then, an emission photograph is taken with a camera or an emission spectrum is analyzed using wave-analysis software in a spectrophotometric system, for example PMA-100 (Hamamatsu Photonics K.K.) or IMUC-7000 (Ohtsuka Denshi K.K.). In this manner, two or more antigens or antibodies can be detected and quantified simultaneously.

### Examples

Hereinafter, the present invention is described specifically by reference to Examples.

### Example 1. Acquisition of biotinated Coleoptera luciferase bL203 and biotinated Coleoptera luciferase bL213

Two oligonucleotides [SLF69 strand (SEQ ID No: 1) and complementary SLF70 strand (SEQ ID No: 2)] coding for biotinated peptide #84 (Met Ala Phe Ser Leu Arg Ser Ile Leu Glu Ala Gln Lys Met Glu Leu Arg Asn Thr Pro Gly Gly Ser) (Lys residue at the 13-position is assumed to be biotinated by the action of biotin holoenzyme synthetase [P. J. Shatz, BIO/TECHNOLOGY, 11, 1138 (1993)] and having restriction enzyme XhoI and MunI sites downstream thereof were synthesized in DNA Model 392 synthesizer (manufactured by Applied Biosystems).

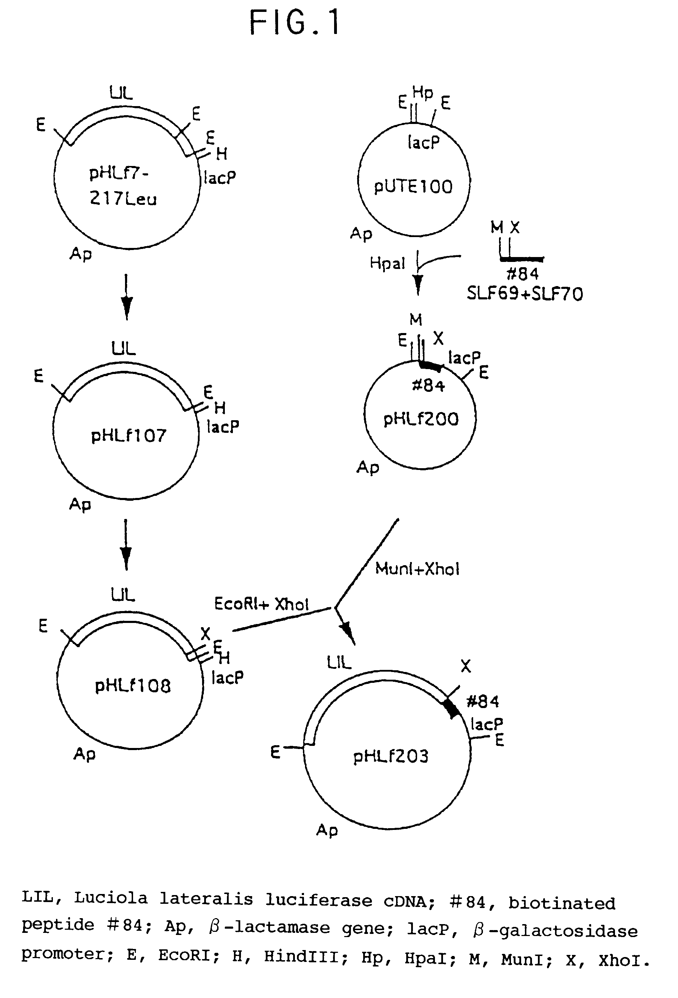

1 pmol of each of oligonucleotides SLF69 and SLF70 was phosphorylated with T4 polynucleotide kinase (produced by Takara Shuzo Co., Ltd.) and the two oligonucleotides were annealed at 90 °C for 10 min. and then at 37 °C for 10 min. Separately, plasmid pUTE100 DNA (described in Japanese Patent Appln. LOP Publication No. 317,055/1993) was cleaved with restriction enzyme HpaI and dephosphorylated with alkaline phosphatase (produced by Takara Shuzo Co., Ltd.). The cleaved plasmid was ligated to the above annealed oligonucleotide with T4 DNA ligase (produced by Takara Shuzo Co., Ltd.) to give recombinant plasmid pHLf200 DNA having the oligonucleotide coding for biotinated peptide #84 inserted into the HpaI site located downstream from the β-galactosidase promoter in plasmid pUTE100 DNA (see FIG. 1).

Separately, single-stranded DNA of recombinant plasmid pHLf7-217 Leu [a plasmid prepared by inserting into plasmid pUC119 a gene for thermostable mutant luciferase from Luciola lateralis in which Ala was replaced by Leu at amino acid 217 (described in Japanese Patent Appln. LOP Publication No. 244,942/1993)] was prepared using helper phage M13 K07 (produced by Takara Shuzo Co., Ltd.). The EcoRI site in the luciferase gene was removed using oligo-nucleotide SLF15 (AGGAATAAAGAACTCTTCACAGTT) and Oligonucleotide-Directed In Vitro Mutagenesis System Version 2 (produced by Amersham) without changing the amino acid sequence of the luciferase gene, whereby plasmid pHLf107 DNA was obtained (see FIG.1). Using oligonucleotide SLF43 (TTCATCGTTCTCGAGGTTTTCCATAGA) (restriction enzyme XhoI site is underlined), the XhoI site was introduced in an analogous manner in the neighborhood of the 5'-terminal of the luciferase gene in recombinant pHLf107 DNA, whereby plasmid pHLf108 was obtained (see FIG. 1).

After pHLf108 was cleaved with restriction enzymes XhoI and EcoRI (both produced by Takara Shuzo Co., Ltd.), a luciferase gene fragment was obtained in agarose gel electrophoresis using a gene clean II kit (produced by BIO101). This fragment was ligated to pHLf200 previously cleaved with XhoI and MunI (both produced by Takara Shuzo Co., Ltd.), to give recombinant plasmid pHLf203 DNA which can initiate the expression of biotinated Coleoptera luciferase bL203 by the β-galactosidase promoter (see FIG. 1).

The same mutation as red-color mutation C-M-5 in Luciola cruciata luciferase (His is replaced by Tyr at amino acid 433; see Japanese Patent Appln. LOP Publication No. 285,683/1991 and US Patent No. 5,219,737) was introduced to biotinated thermostable Coleoptera luciferase bL203 derived from Luciola lateralis emitting yellow light (see Japanese Patent Application No. 98857/1995 and US Patent Application No. 08/460,934).

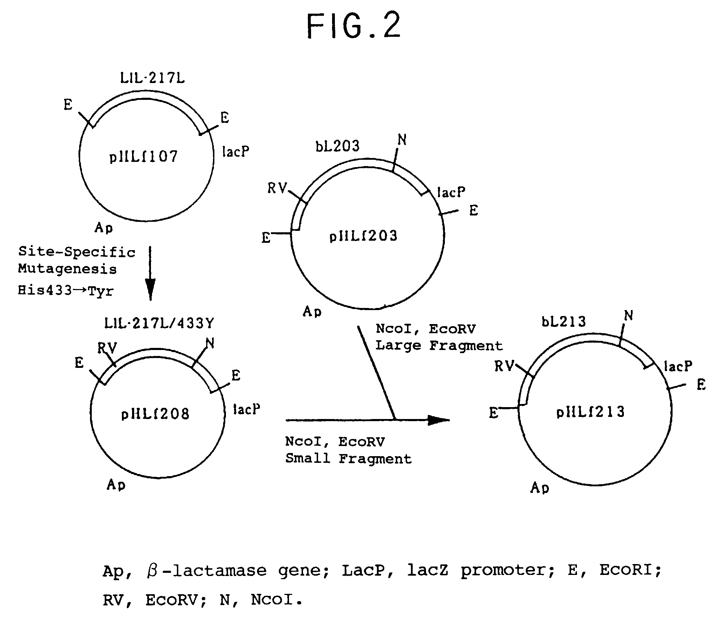

Single-stranded plasmid pHLf107 was prepared from E. coli CJ236 [pHLf107] (E. coli CJ236 is available from Bio-Rad). This plasmid pHLf107 was used to prepare plasmid pHLf208 coding for thermostable Luciola lateralis luciferase in which His is replaced by Tyr at amino acid 433 by use of Muta-Gene in vitro Mutagenesis Kit (Bio-Rad) with oligonucleotide SLF85 (ATAAAGAAATATTTTTCTTCA) synthesized in DNA Model 392 synthesizer (Applied Biosystems) (see FIG. 2).

Plasmid pHLf208, and plasmid pHLf203 containing the biotinated luciferase bL203 gene, were cleaved respectively with restriction enzymes NcoI and EcoRV and subjected to agarose gel electrophoresis, and the smaller fragment from pHLf208 and the larger fragment from pHLf203 were recovered with Gene Clean II Kit (BIO101), and these fragments were ligated to give plasmid pHLf213 containing the gene coding for biotinated Coleoptera luciferase bL213 in which His was replaced by Try at the 453-position (corresponding to the 433-position in luciferases from Luciola cruciata and Luciola lateralis)(see FIG. 2).

E. coli JM101 [pHLf213] carrying plasmid pHLf213, which is deposited as FERM BP-5484 with the National Institute of Bioscience and Human-Technology, Agency of Industrial Science and Technology, Japan, was plated onto a sterilized nitrocellulose filter and incubated for 16 hours at 37 °C in LB agar medium [1 % Bacto-Trypton (DIFCO), 0.5 % yeast extract, 0.5 % NaCl, 1.4 % agar] supplemented with 50 µ g/ml ampicillin and 1 mM isopropyl-β -galactoside (IPTG), and then the nitrocellulose filter was immersed in a luminescent substrate solution (0.5 mM luciferin, 100 mM sodium citrate, pH 5.0) and the emission of an orange light by E. coli JM101 [pHLf213] was confirmed by observation in the dark. That is, the introduction of the same mutation as red-color mutation C-M-3 in the Luciola cruciata luciferase into biotinated luciferase bL203 gave biotinated luciferase bL213 capable of emitting an orange light by catalyzing luciferin.

E. coli JM101 [pHLf203] (FERM BP-5052) and E. coli JM101 [pHLf213] were cultured respectively in the following manner, and biotinated luciferase bL203 and biotinated luciferase bL213 which could emit yellow and orange lights respectively by catalyzing luciferin were purified.

E. coli JM101 [pHLF203] or E. coli JM101 [pHLF213] was cultured at 30 °C with stirring at 120 r.p.m. for 5 hours in 1 liter of LB medium (1 % Bacto-trypton, 0.5 % yeast extract, 0.5 % sodium chloride) containing 0.2 mM isopropyl-β-thiogalactoside (IPTG), 50 µ g/ml ampicillin and 10 µ g/ml D-biotin. The microorganism was recovered by centrifugation at 5,000 r.p.m. for 5 min. and suspended in 100 ml buffer [25 mM tris(hydroxymethyl) aminomethane (TRIS), 1 mM ethylenediaminetetraacetic acid (EDTA), 10 % saturated ammonium sulfate, 1 mg/ml lysozyme, pH 7.8]. The microorganism was lysed by being frozen and melted 3 times and centrifuged at 12,000 r.p.m. for 5 min. to give a supernatant of the disrupted microorganism. The purification of bL203 or bL213 from this supernatant was conducted according to the method described by Kajiyama et al. [N. Kajiyama et al., Biochim. Biophys. Acta, 1120, 228 (1992)].

### 2. Preparation of biotinated luciferase/streptavidin/biotinated antibody complexes

To detect mouse IgG₁ and mouse IgM simultaneously with biotinated luciferases capable of emitting different colors of light, the two kinds of biotinated luciferase were bound respectively to biotinated antibodies via streptavidin.

bL203 and bL213 capable of emitting yellow and orange lights respectively, obtained in item 1, were used as biotinated luciferases. As their corresponding antibodies, biotinated anti-mouse IgG₁ rat monoclonal antibody clone LO-MG1-2 (produced by Cybus Bioscience) and biotinated anti-mouse IgM rabbit polyclonal antibody (produced by Zymed) were used respectively. 1 mg/ml biotinated luciferase and 1 mg/ml streptavidin were mixed in an equal volume, then left at room temperature for 15 min. and subjected to gel filtration on a Superdex 200 HR10/30 column (Pharmacia Biotech). The mobile phase was buffer A (50 mM sodium phosphate, 200 mM NaCl, 10 % glycerin, pH 7.4), and the sample was applied in an amount of 50 µ l and separated at a flow rate of 0.5 ml/min. A fraction containing a complex of the biotinated luciferase/streptavidin (1 : 1) was identified by its absorption at 280 nm, its luciferase activity and SDS polyacrylamide gel electrophoresis. This complex was concentrated to 0.4 mg/ml by use of Centriprep (produced by Amicon), and 1 mg/ml biotinated antibody was mixed with it in an equal volume. In this manner, fractions containing complexes of the biotinated luciferase/ streptavidin/biotinated antibody (i.e. bL203/streptavidin/ biotinated IgG₁ antibody complex, referred to as complex A, and of bL213/streptavidin/biotinated anti-IgM antibody complex, referred to as complex B) were respectively obtained.

### 3. Dot blots with the biotinated luciferase/streptavidin/ biotinated antibody complexes

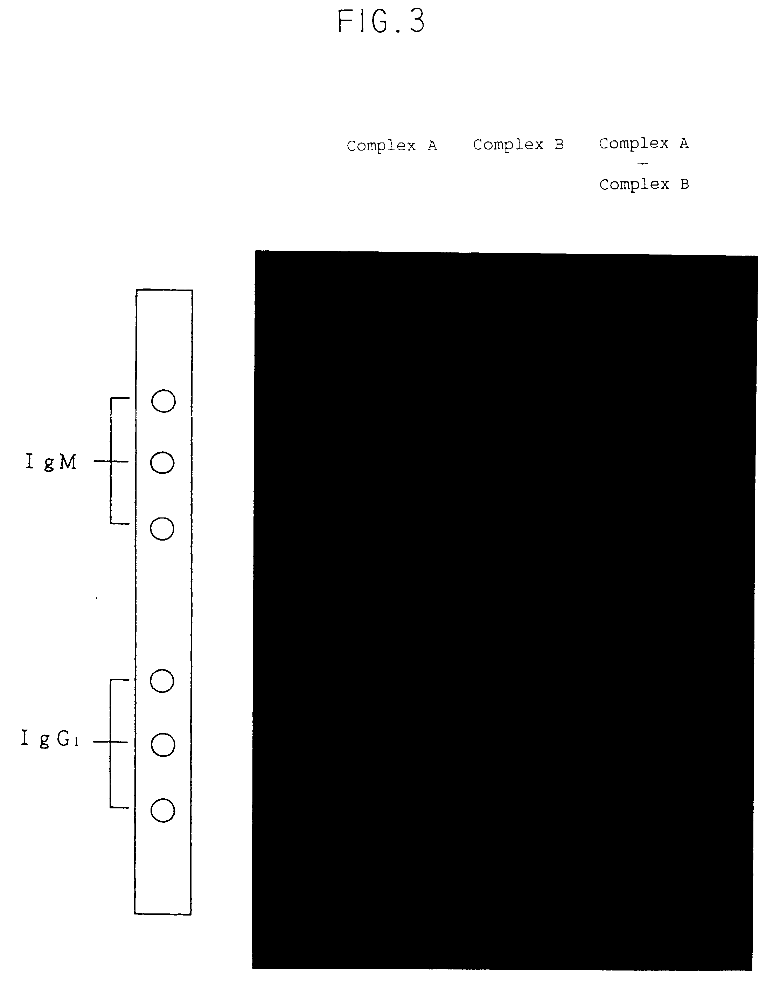

Simultaneous detection of mouse IgG₁ and mouse IgM was attempted using the two kinds of biotinated luciferase/ streptavidin/biotinated antibody complex obtained in item 2. PVDF membrane Immobilon-PSQ (Millipore) was rendered hydrophilic by treatment and 4 µ l each of 62.5 µ g/ml IgG₁ and IgM were dropped on the PVDF membrane. The PVDF membrane was prepared in triplicate and washed for 5 min. 3 times. with 10 % Block Ace (Dainippon Pharmaceutical Co., Ltd.) containing 0.05 % Tween 20 and blocked with 100 % Block Ace at room temperature for 2 hours. The respective membranes were washed similarly, and complex A was added to one membrane in an amount of 1×10⁻¹² mol/ml (in terms of bL203 emission), and complex B to another membrane in 2×10⁻¹¹ mol/ml (in bL213 emission), and both of them to the last one, and the complex was allowed to immerse the membrane at room temperature for 1 hour. After the complex was removed, the membranes were washed 4 times and immersed in buffer B (300 mM magnesium sulfate, 50 mM HEPES, pH 7.5) for 5 minutes. Buffer B was removed, and another buffer B containing 1.4 mM luciferin and 40 mM ATP was sprayed on the membranes in the dark and a photograph of their emission was taken with an Ektachrome professional P1600 film (Kodak). They were exposed for 20 min. at F2.5 (FIG. 3).

As shown in FIG. 3, when complex A only was added, the IgG₁ spots only emitted yellow color of light which is the color of bL203 emission, and when complex B only was added, the IgM spots only emitted orange color of light which is the color of bL213 emission. Further, when the two complexes were added, the IgG₁ spots emitted the same yellow color of light as in the case where only complex A was added, while the IgM spots emitted the same orange color of light as in the case where only complex B was added. That is, the emission was observed as expected without the cross-reaction therebetween. It was found that two kinds of antigen can be detected simultaneously by this method.

E.coli JM101 [pHLf203] carrying plasmid pHLf203 is deposited as FERM BP-5052 with the National Institute of Bioscience and Human-Technology, Agency of Industrial Science and Technology, Japan.

SEQUENCE TABLE

Sequence No: 1

Length: 85

Sequence Type: nucleic Acid

Strand Form: single

Topology: linear

Nature: synthetic DNA

Feature: oligonucleotide SLF69

Sequence:

ATGGCATTTTCATTACGTTCTATTCTTGAAGCTCAAAAAATGGAATTACGTAACACTCCA 60

GGAGGTAGTCTCGAGGCTACAATTG 85

Sequence No: 2

Length: 85

Sequence Type: nucleic acid

Strand Form: single

Topology: linear

Nature: synthetic DNA

Feature: oligonucleotide SLF70

Sequence:

CAATTGTAGCCTCGAGACTACCTCCTGGAGTGTTACGTAATTCCATTTTTTGAGCTTCAA 60

GAATAGAACGTAATGAAAATGCCAT 85

## Claims

1. An immunoassay using an enzyme as a label, wherein two or more kinds of luciferase are used as the label to determine two or more substances.

2. An immunoassay according to claim 1, wherein each luciferase emits light of a different respective wavelength when acting upon a luciferin substrate.

3. An immunoassay according to claim 2 wherein the different wavelengths of light differ from each other by 2 to 20nm.

4. An immunoassay according to claim 3 wherein the different wavelengths of light differ from each other by 3 to 17nm.

5. An immunoassay according to any one of claims 2 to 4 wherein each luciferase emits light at a different respective wavelength selected from 560nm, 595nm, 607nm, 609nm and 612nm.

6. An immunoassay according to any one of the preceding claims wherein the luciferases are selected from wild and mutant *Coleoptera* luciferases.

7. An immunoassay according to claim 6 wherein the luciferases are selected from
(i) the luciferases of *Luciola cruciata, Luciola lateralis* and *Photinus pyralis,*
(ii) a mutant luciferase having the amino acid sequence of the luciferase of *Luciola cruciata* in which serine is replaced by asparagine at amino acid 286
(iii) a mutant luciferase having the amino acid sequence of the luciferase of *Luciola cruciata* in which glycine is replaced by serine at amino acid 326
(iv) a mutant luciferase having the amino acid sequence of the luciferase of *Luciola cruciata* in which histidine is replaced by tyrosine at amino acid 433; and
(v) a mutant luciferase having the amino acid sequence of the luciferase of *Luciola cruciata* in which proline is replaced by serine at amino acid 452.

8. An immunoassay according to any one of the preceding claims which comprises:
(i) immobilising onto a carrier two or more substances to be determined, the said substances being selected from antigens and antibodies;
(ii) contacting the said immobilised substances with corresponding antibodies or antigens labelled with different luciferases;
(iii) adding a substrate for the luciferases; and
(iv) detecting the light emitted by the luciferases.

9. An immunoassay according to any one of claims 1 to 7 which comprises:
(i) immobilising onto a solid support one or more antibodies or antigens to which the antigens or antibodies to be determined can bind;
(ii) contacting the immobilised antibodies or antigens with the antigens or antibodies to be determined thereby to immobilise the antigens or antibodies to be determined;
(iii) contacting the immobilised antigens or antibodies to be determined with corresponding antibodies or antigens labelled with different luciferases;
(iv) adding a substrate for the luciferases; and
(v) detecting the light emitted by the luciferases.

10. An immunoassay according to claim 8 or 9 wherein the quantity of light and/or the wavelength of light emitted from each luciferase is determined.


SEQUENCE LISTING

(1) GENERAL INFORMATION:

(i) APPLICANT:
(A) NAME: KIKKOMAN CORPORATION
(B) STREET: 339, NODA, NODA-SHI
(C) CITY: CHIBA-KEN
(E) COUNTRY: JAPAN
(F) POSTAL CODE (ZIP): 278

(ii) TITLE OF INVENTION: MULTIPLE IMMUNOASSAYS

(iii) NUMBER OF SEQUENCES: 5

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97302099.3

(2) INFORMATION FOR SEQ ID NO: 1:

(i) SEQUENCE CHARACTERISTICS:
(A) LENGTH: 85 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 1:

ATGGCATTTT CATTACGTTC TATTCTTGAA GCTCAAAAAA TGGAATTACG TAACACTCCA 60

GGAGGTAGTC TCGAGGCTAC AATTG 85

(2) INFORMATION FOR SEQ ID NO: 2:

(i) SEQUENCE CHARACTERISTICS:
(A) LENGTH: 85 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 2:

CAATTGTAGC CTCGAGACTA CCTCCTGGAG TGTTACGTAA TTCCATTTTT TGAGCTTCAA 60

GAATAGAACG TAATGAAAAT GCCAT 85

(2) INFORMATION FOR SEQ ID NO: 3:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 3:

AGGAATAAAG AACTCTTCAC AGTT 24

(2) INFORMATION FOR SEQ ID NO: 4:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 4:

TTCATCGTTC TCGAGGTTTT CCATAGA 27

(2) INFORMATION FOR SEQ ID NO: 5:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 5:

ATAAAGAAAT ATTTTTCTTC A 21